# EUROPÄISCHE PATENTANMELDUNG

(11) **EP 4 756 426 A1**
(43) Veröffentlichungstag der Anmeldung: **10.06.2026**
(21) Anmeldenummer: 24217961.2
(22) Anmeldetag: 06.12.2024
(51) Int. Cl.: G01N 33/34, H01M 4/00

(54) **VERFAHREN UND VORRICHTUNG ZUR ANALYSE EINER BESCHICHTUNG AUF EINER BESCHICHTETEN MATERIALBAHN IN BEZUG AUF DARIN ENTHALTENE FEUCHTIGKEIT**

(71) Anmelder: Jagenberg Converting Solutions GmbH, 46397 Bocholt (DE)
(72) Erfinder: Eggerath, Daniel, 22527 Hamburg (DE)
(74) Vertreter: Fink Numrich Patentanwälte PartmbB

(57) **Zusammenfassung**

Die Erfindung betrifft ein Verfahren zur Analyse einer Beschichtung (1a) auf einer beschichteten Materialbahn (1) in Bezug auf darin enthaltene Feuchtigkeit, bei dem:
a) eine Messkammer (3) mit Messkammerluft (AI) befüllt wird, welche eine vorgegebene relative Luftfeuchtigkeit (HU) und eine vorgegebene Temperatur (TP) am Anfang eines Zeitintervalls (IN) aufweist, das nach dem Befüllen der Messkammer (3) beginnt;
b) durch die befüllte Messkammer (3) die beschichtete Materialbahn (1) transportiert wird und während des Transports der Materialbahn (1) ein oder mehrere Parameter der Messkammerluft (AI) umfassend die relative Luftfeuchtigkeit (HU') der Messkammerluft (AI) zumindest am Ende des Zeitintervalls (IN) gemessen wird;
c) aus der Veränderung der relativen Luftfeuchtigkeit der Messkammerluft (AI) innerhalb des Zeitintervalls (IN) mithilfe der vorgegebenen Temperatur (TP) der Messkammerluft (AI) und/oder mithilfe einer in Schritt b) gemessenen Temperatur (TP') der Messkammerluft (AI) am Ende des Zeitintervalls (IN) eine Feuchtigkeitsinformation (HI) ermittelt wird, welche den Wassergehalt in der Beschichtung (1a) der beschichteten Materialbahn (1) beschreibt.

## Beschreibung

Die Erfindung betrifft ein Verfahren und eine Vorrichtung zur Analyse einer Beschichtung auf einer beschichteten Materialbahn in Bezug auf darin enthaltene Feuchtigkeit.

In einer Vielzahl von technischen Anwendungsgebieten werden Materialbahnen in einem Fertigungsprozess mit einer Beschichtung versehen. Häufig besteht dabei die Notwendigkeit, die Feuchtigkeit in der Beschichtung möglichst gering zu halten. Unter Feuchtigkeit ist hier und im Folgenden der Wassergehalt in der entsprechenden Beschichtung zu verstehen. Der Wassergehalt wird vorzugsweise über einen relativen Anteil an Wasser in Bezug auf die trockene Beschichtung ohne Wasser beschrieben, z.B. angegeben durch ppm (ppm = parts per million).

Um die Restfeuchte in der Beschichtung einer Materialbahn zu reduzieren, werden häufig Trocknungsanlagen eingesetzt, durch welche die beschichtete Materialbahn transportiert wird und dabei Trocknungsluft ausgesetzt wird. Solche Trocknungsanlagen kommen beispielsweise bei beschichteten Materialbahnen für Batterieelektroden zum Einsatz.

Um den Wassergehalt von beschichteten Materialbahnen zu messen, sind aus dem Stand der Technik Spektrometer bekannt, welche die Feuchtigkeit der Beschichtung mittels Licht, beispielsweise im nahen Infrarotbereich, bestimmen. Diese spektroskopische Feuchtigkeitsmessung ist jedoch aufwändig und teuer.

Aufgabe der Erfindung ist es, ein Verfahren und eine Vorrichtung zur Analyse einer Beschichtung auf einer beschichteten Materialbahn zu schaffen, mit denen einfach und kostengünstig der Grad an Feuchtigkeit in der Beschichtung ermittelt werden kann.

Diese Aufgabe wird durch das Verfahren gemäß Patenanspruch 1 bzw. die Vorrichtung gemäß Patentanspruch 14 gelöst. Weiterbildungen der Erfindung sind in den abhängigen Ansprüchen definiert.

In einem Schritt a) des erfindungsgemäßen Verfahrens wird eine Messkammer mit Messkammerluft befüllt, welche eine vorgegebene relative Luftfeuchtigkeit und eine vorgegebene Temperatur am Anfang eines Zeitintervalls aufweist, das nach dem Befüllen der Messkammer beginnt. Die relative Luftfeuchtigkeit und die Temperatur der Messkammerluft am Anfang des Zeitintervalls sind somit vorbekannt. Sie können z.B. mit einer Sensorik in der Messkammer bestimmt bzw. überwacht werden. Je nach Ausgestaltung kann der Anfang des obigen Zeitintervalls unterschiedlich festgelegt werden. Beispielsweise kann sich das Zeitintervall unmittelbar an die Befüllung der Messkammer mit Messkammerluft anschließen. Ferner kann das Zeitintervall auch erst dann beginnen, wenn sich ein Temperaturgleichgewicht in der Messkammer eingestellt hat, d.h. ab einem Zeitpunkt, zu dem die Temperatur in der Messkammer im Wesentlichen konstant bleibt.

In einer Variante ist die zugeführte Messkammerluft Raumluft aus der Umgebung der Messkammer. Nichtsdestotrotz ist es auch möglich, dass die Raumluft oder Luft aus einer anderen Quelle vorkonditioniert wird, um sie auf eine bestimmte relative Luftfeuchtigkeit und Temperatur einzustellen. Die vorkonditionierte Luft wird dann als Messkammerluft der Messkammer zugeführt.

In einem Schritt b) des erfindungsgemäßen Verfahrens wird durch die befüllte Messkammer die beschichtete Materialbahn transportiert, wobei während des Transports der Materialbahn ein oder mehrere Parameter der Messkammerluft umfassend die relative Luftfeuchtigkeit der Messkammerluft zumindest am Ende des Zeitintervalls gemessen werden. Gegebenenfalls kann in dem Zeitintervall auch mehrmals die relative Luftfeuchtigkeit der Messkammerluft gemessen werden, d.h. die relative Luftfeuchtigkeit der Messkammerluft wird nicht nur am Ende des Zeitintervalls, sondern auch davor gemessen.

In einem Schritt c) des erfindungsgemäßen Verfahrens wird aus der Veränderung der relativen Luftfeuchtigkeit der Messkammerluft innerhalb des Zeitintervalls mithilfe der vorgegebenen Temperatur der Messkammerluft und/oder mithilfe einer in Schritt b) gemessenen Temperatur der Messkammerluft am Ende des Zeitintervalls eine Feuchtigkeitsinformation ermittelt, welche den Wassergehalt in der Beschichtung der beschichteten Materialbahn beschreibt. In einer Variante gibt die Feuchtigkeitsinformation unmittelbar den Wassergehalt in der Beschichtung an. Nichtsdestotrotz kann die Feuchtigkeitsinformation den Wassergehalt auch auf andere Weise, z.B. basierend auf dem Überschreiten bzw. Unterschreiten eines Schwellwerts, beschreiben, wie weiter unten erläutert wird. Die gemessene Temperatur der Meskammerluft am Ende des Zeitintervalls stellt einen entsprechenden (gemessenen) Parameter der Messkammerluft dar. Vorzugsweise wird diese Temperatur auch immer dann gemessen, wenn in Schritt b) die relative Luftfeuchtigkeit gemessen wird.

Das erfindungsgemäße Verfahren beruht auf der Erkenntnis, dass sich aus der Veränderung der relativen Luftfeuchtigkeit von Messkammerluft, die durch den Austausch von Wassermolekülen zwischen der Messkammerluft und der Beschichtung der Materialbahn verursacht ist, Rückschlüsse auf den Wassergehalt in der Beschichtung ziehen lassen und demzufolge eine Feuchtigkeitsinformation in Bezug auf den Wassergehalt in der Beschichtung ermittelt werden kann.

Das erfindungsgemäße Verfahren kann gegebenenfalls in vorbestimmten Zeitabständen wiederholt werden, wobei hierfür die vorhandene Messkammerluft aus der Messkammer abgelassen wird und die Messkammer neu mit Messkammerluft mit vorgegebener relativer Luftfeuchtigkeit und vorgegebener Temperatur befüllt wird.

In einer bevorzugten Variante des erfindungsgemäßen Verfahrens bleibt die Temperatur der Messkammerluft innerhalb des Zeitintervalls im Wesentlichen konstant. Dies kann z.B. durch eine geeignete Wahl des Anfangs des Zeitintervalls oder durch eine Einrichtung zum Beheizen und/oder Kühlen der Messkammerluft erreicht werden.

In einer besonders bevorzugten Ausführungsform ergibt sich die Feuchtigkeitsinformation aus zumindest einer Sorptionsisotherme und/oder zumindest einer Desorptionsisotherme. Die zumindest eine Sorptionsisotherme bzw. die zumindest eine Desorptionsisotherme repräsentiert einen Gleichgewichtszustand zwischen dem Wassergehalt in der Beschichtung und dem Wassergehalt in umgebender Luft mit der vorgegebenen Temperatur und/oder der gemessenen Temperatur am Ende des Zeitintervalls. In diesem Gleichgewichtszustand werden keine Wassermoleküle mehr zwischen Beschichtung und umgebender Luft ausgetauscht. Dabei beschreibt die entsprechende Sorptionsisotherme im Falle einer vor Erreichen des Gleichgewichtzustands stattgefundenen Sorption (d.h. Adsorption und ggf. auch Absorption) von Wasser in die Beschichtung aus der umgebenden Luft mit der vorgegebenen bzw. gemessenen Temperatur, d.h. bei Rückbefeuchtung der Beschichtung über Wasser aus der umgebenden Luft, die Abhängigkeit des Wassergehalts in der Beschichtung von der relativen Luftfeuchtigkeit der umgebenden Luft mit der vorgegebenen bzw. gemessenen Temperatur im Gleichgewichtszustand. Sorptionsisothermen werden häufig auch als Adsorptionsisothermen bezeichnet, auch wenn bei der Sorption neben einer Adsorption eine Absorption von Wasser stattfinden kann. Die entsprechende Desorptionsisotherme beschreibt im Falle einer vor Erreichen des Gleichgewichtszustands stattgefundenen Desorption von Wasser aus der Beschichtung in die umgebende Luft mit der vorgegebenen bzw. gemessenen Temperatur, d.h. bei Abgabe von Wasser aus der Beschichtung an die umgebende Luft, die Abhängigkeit des Wassergehalts in der Beschichtung von der relativen Luftfeuchtigkeit der umgebenden Luft mit der vorgegebenen bzw. gemessenen Temperatur im Gleichgewichtszustand.

Sorptionsisothermen und Desorptionsisothermen und deren Messung sind hinlänglich aus dem Stand der Technik bekannt (siehe beispielsweise Druckschriften [1] und [2]). In der soeben beschriebenen Ausführungsform der Erfindung wird auf eine vorab gemessene oder berechnete Sorptions- bzw. Desorptionsisotherme für die entsprechende Beschichtung bei der vorgegebenen bzw. am Ende des Zeitintervalls gemessenen Temperatur der umgebenden Luft zurückgegriffen. Für bestimmte Beschichtungen können die Sorptionsisotherme und die Desoptionsisotherme zusammenfallen.

In einer weiteren bevorzugten Variante des erfindungsgemäßen Verfahrens wird in Schritt c) bei Abnahme der relativen Luftfeuchtigkeit der Messkammerluft ausgehend von der vorgegebenen relativen Luftfeuchtigkeit innerhalb des Zeitintervalls als zumindest ein Teil der Feuchtigkeitsinformation der Wassergehalt in der Beschichtung ermittelt, der sich aus der Sorptionsisotherme für die gemessene relative Luftfeuchtigkeit der Messkammerluft und die gemessene Temperatur der Messkammerluft am Ende des Zeitintervalls ergibt, wobei die Länge des Zeitintervalls vorzugsweise derart gewählt ist, dass davon ausgegangen werden kann, das sich der Gleichgewichtszustand zwischen dem Wassergehalt in der Beschichtung und dem Wassergehalt in der Messkammerluft eingestellt hat. Diese Variante der Erfindung kommt dann zum Einsatz, wenn eine Rückbefeuchtung der Beschichtung aufgrund der Abnahme der relativen Luftfeuchtigkeit der Messkammerluft festgestellt wird, so dass die Abhängigkeit des Wassergehalts der Beschichtung von der relativen Luftfeuchtigkeit der Messkammerluft im Gleichgewichtszustand über die Sorptionsisotherme beschrieben wird.

In einer weiteren Ausführungsform des erfindungsgemäßen Verfahrens wird in Schritt c) bei Zunahme der relativen Luftfeuchtigkeit der Messkammerluft ausgehend von der vorgegebenen relativen Luftfeuchtigkeit innerhalb des Zeitintervalls als zumindest ein Teil der Feuchtigkeitsinformation der Wassergehalt in der Beschichtung ermittelt, der sich aus der Desorptionsisotherme für die gemessene relative Luftfeuchtigkeit der Messkammerluft und die gemessene Temperatur der Messkammerluft (AI) am Ende des Zeitintervalls ergibt, wobei die Länge des Zeitintervalls vorzugsweise derart gewählt ist, dass davon ausgegangen werden kann, das sich der Gleichgewichtszustand zwischen dem Wassergehalt in der Beschichtung und dem Wassergehalt in der Messkammerluft eingestellt hat. Diese Variante der Erfindung wird dann verwendet, wenn Wasser von der Beschichtung in die Messkammerluft abgegeben wird, so dass die Abhängigkeit der relativen Luftfeuchtigkeit der Messkammerluft von dem Wassergehalt in der Beschichtung über die Desorptionsisotherme beschrieben wird.

Sollte sich die relative Luftfeuchtigkeit der Messkammerluft ausgehend von der vorgegeben relativen Luftfeuchtigkeit innerhalb des Zeitintervalls nicht verändern, kann der Wassergehalt entsprechend den beiden oben beschriebenen Varianten entweder mittels der Sorptionsisotherme oder mittels der Desorptionsisotherme bestimmt werden. In der Regel sind die Abweichungen zwischen Sorptionsisotherme und Desorptionsisotherme gering.

In einer weiteren bevorzugten Ausführungsform ist ein Schwellwert für den Wassergehalt in der Beschichtung festgelegt. Dieser Schwellwert, der beispielsweise zwischen 100 ppm und 200 ppm liegt, gibt in der Regel den maximal zulässigen Wassergehalt in der Beschichtung an, d.h. bei Überschreiten dieses Schwellwerts ist die Beschichtung noch nicht ausreichend trocken. Dabei wird die vorgegebene Luftfeuchtigkeit und die vorgegebene Temperatur der Messkammerluft derart eingestellt, dass sich aus der Sorptionsisotherme, welche den Gleichgewichtszustand zwischen dem Wassergehalt in der Beschichtung und dem Wassergehalt in umgebender Luft mit der vorgegebenen Temperatur beschreibt, für die vorgegebene Luftfeuchtigkeit der umgebenden Luft der Schwellwert ergibt. Als zumindest ein Teil der Feuchtigkeitsinformation wird ermittelt, dass der Wassergehalt in der Beschichtung kleiner gleich dem Schwellwert ist, wenn die relative Luftfeuchtigkeit der Messkammerluft ausgehend von der vorgegebenen Luftfeuchtigkeit innerhalb des vorgegebenen Zeitintervalls abnimmt oder konstant bleibt, und dass der Wassergehalt in der Beschichtung größer als der Schwellwert ist, wenn die relative Luftfeuchtigkeit der Messkammerluft ausgehend von der vorgegebenen relativen Luftfeuchtigkeit innerhalb des Zeitintervalls zunimmt. Bei dieser Variante kann das Zeitintervall relativ kurz eingestellt werden, da lediglich detektiert werden muss, dass eine Zunahme bzw. Abnahme der relativen Luftfeuchtigkeit der Messkammerluft stattfindet. Eine Feuchtigkeitsinformation in Bezug auf die Beschichtung kann somit in kurzer Zeit ermittelt werden.

Je nach Ausgestaltung kann das Zeitintervall, an dessen Ende die relative Luftfeuchtigkeit der Messkammerluft gemessen wird, unterschiedlich festgelegt sein. In einer Variante ist das Zeitintervall fest vorgegeben und liegt vorzugsweise zwischen 10 Sekunden und 10 Minuten, besonders bevorzugt zwischen 20 Sekunden und 1 Minute.

In einer weiteren Ausführungsform wird das Zeitintervall variabel in Abhängigkeit von der sich verändernden relativen Luftfeuchtigkeit der Messkammerluft bestimmt. Das Zeitintervall wird dabei dann beendet, wenn die Veränderung der relativen Luftfeuchtigkeit der Messkammerluft unterhalb einer vorgegebenen Schwelle liegt, so dass davon ausgegangen werden kann, dass sich der Gleichgewichtszustand zwischen dem Wassergehalt in der Beschichtung und dem Wassergehalt in der Messkammerluft eingestellt hat. Die Veränderung der relativen Luftfeuchtigkeit kann beispielsweise als Gradient des zeitlichen Verlaufs der Luftfeuchtigkeit oder als Differenz von zwei Luftfeuchtigkeiten in einer kurzen Zeitspanne bestimmt werden.

In einer weiteren bevorzugten Ausführungsform zirkuliert die Messkammerluft in der Messkammer, wodurch ein guter Austausch von Wassermolekülen zwischen der Beschichtung der Materialbahn und der Messkammerluft gewährleistet ist.

In einer weiteren bevorzugten Variante steht die Messkammerluft gegenüber der Luft in der Umgebung der Messkammer unter Überdruck. Hierdurch wird eine schnelle Zufuhr und Abfuhr von Messkammerluft zur Durchführung des erfindungsgemäßen Verfahrens ermöglicht. Gleichzeitig gewährleistet ein Überdruck innerhalb der Messkammer, dass aus der Umgebung keine Fremdluft in die Messkammer eintritt. Vorzugsweise wird der Überdruck konstant gehalten, was mit einer geeigneten Druckmessung überwacht werden kann. Ein konstanter Überdruck innerhalb der Messkammer gewährleistet ein konstantes Luftvolumen in der Messkammer.

Um in möglichst kurzer Zeit die Einstellung des Gleichgewichtszustand zwischen dem Wassergehalt in der Beschichtung und dem Wassergehalt in der Messkammerluft herzustellen, sollten die Abmessungen der Messkammer nicht zu groß gewählt werden. In einer bevorzugten Variante hat die Messkammer eine vorzugsweise konstante Länge in Transportrichtung der Materialbahn zwischen 20 cm und 40 cm, vorzugsweise 30 cm, und/oder eine vorzugsweise konstante Höhe in Richtung senkrecht zur Ebene der Materialbahn zwischen 3 cm und 5 cm, vorzugsweise 4 cm, und/oder eine vorzugsweise konstante Breite senkrecht zur Transportrichtung der Materialbahn zwischen 200 mm und 1600 mm. Vorzugsweise ist der Abstand der Oberseite der Messkammer zur Materialbahn genauso groß wie der Abstand der Unterseite der Messkammer zur Materialbahn. Das heißt, die Materialbahn wird mittig durch die Messkammer hindurchgeführt.

Ein besonders bevorzugter Anwendungsfall des erfindungsgemäßen Verfahrens ist die Analyse der Beschichtung einer beschichteten Elektrodenmaterialbahn für eine Batterie. Bei dem Elektrodenmaterial kann es sich um die Anode oder die Kathode der Batterie handeln, die im Herstellungsprozess mit einem sog. Slurry beschichtet wird.

In einer weiteren bevorzugten Ausführungsform wird das erfindungsgemäße Verfahren nach einem Trocknungsprozess der beschichteten Materialbahn durchgeführt. Gegebenenfalls ist dabei möglich, dass Parameter des Trocknungsprozesses verändert werden, wenn mit dem erfindungsgemäßen Verfahren eine zu große Feuchtigkeit in der Beschichtung der Materialbahn festgestellt wird.

Neben dem oben beschriebenen Verfahren betrifft die Erfindung eine Vorrichtung zur Analyse einer Beschichtung auf einer beschichteten Materialbahn in Bezug auf darin enthaltene Feuchtigkeit, wobei die Vorrichtung zur Durchführung des erfindungsgemäßen Verfahrens bzw. einer oder mehrerer bevorzugter Varianten des erfindungsgemäßen Verfahrens eingerichtet ist. Mit anderen Worten enthält die Vorrichtung eine entsprechende Messkammer, ein Mittel zum Befüllen und vorzugweise auch Entleeren der Messkammer mit Messkammerluft, einen Feuchtigkeitssensor und optional einen Temperatursensor, um die relative Luftfeuchtigkeit und die Temperatur der Messkammerluft zu messen, sowie eine Auswerteeinheit, welche die Feuchtigkeitsinformation aus der Veränderung der relativen Luftfeuchtigkeit der Messkammerluft mithilfe der vorgegebenen Temperatur der Messkammerluft und/oder mithilfe der gemessenen Temperatur der Messkammerluft am Ende des Zeitintervalls ermittelt.

Ausführungsbeispiele der Erfindung werden nachfolgend anhand der beigefügten Figuren detailliert beschrieben.

Es zeigen:
- Fig. 1: eine schematische Schnittdarstellung einer Messvorrichtung zur Durchführung einer Ausführungsform des erfindungsgemäßen Verfahrens;
- Fig. 2: ein Diagramm, welches eine berechnete Sorptionsisotherme und eine berechnete Desorptionsisotherme für die Beschichtung einer Elektrodenmaterialbahn wiedergibt; und
- Fig. 3: ein Diagramm, welches die zeitliche Veränderung der relativen Luftfeuchtigkeit in einer Messkammer in Abhängigkeit von unterschiedlichen Wassergehalten der Beschichtung der Materialbahn unter Verwendung der Sorptionsisotherme der Fig. 2 wiedergibt.

Nachfolgend wird eine Variante des erfindungsgemäßen Verfahrens anhand einer beschichteten Materialbahn in der Form einer Elektrodenmaterialbahn beschrieben. Nichtsdestotrotz kann die Erfindung auch für beliebige andere Materialbahnen eingesetzt werden.

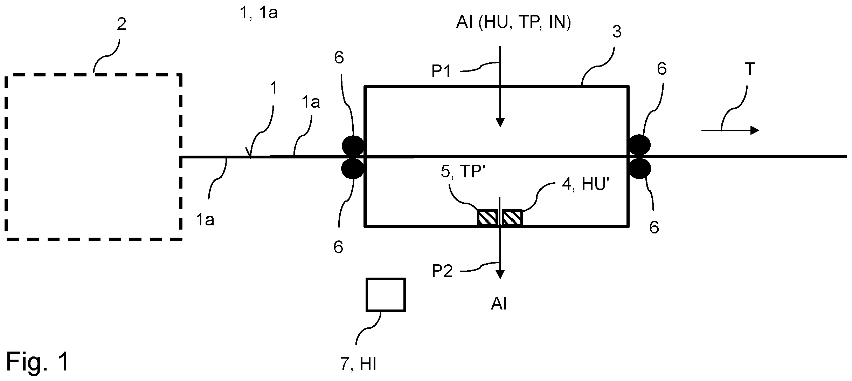

In Fig. 1 ist die Materialbahn mit Bezugszeichen 1 bezeichnet. Diese Materialbahn weist auf ihrer Oberseite und Unterseite jeweils eine Beschichtung 1a auf, die im Rahmen eines an sich bekannten Herstellungsprozesses für Batterieelektroden aufgebracht wurde. Je nach Ausgestaltung kann es sich bei der Materialbahn 1 um beschichtetes Anodenmaterial oder Kathodenmaterial handeln. Die Kathode ist beispielsweise eine Aluminiumfolie, auf die eine Beschichtung aufgebracht ist, welche Lithium-Nickel-Mangan-Kobalt-Oxide als Aktivmaterial enthält. Die Anode ist beispielsweise eine Kupferfolie, auf die eine Beschichtung aufgebracht ist, welche Graphit als Aktivmaterial enthält.

In dem Szenario der Fig. 1 wurde das Materialbahn 1 kalandriert (d.h. mittels Walzen verpresst) und anschließend in einem Trockner getrocknet, um unerwünschtes Wasser aus der Beschichtung 1a der Materialbahn 1 zu entfernen. Fig. 1 zeigt schematisch den Ausgang 2 des entsprechenden Trockners. Die Materialbahn 1 wird nach dem Trocknen in die durch den Pfeil T angedeutete Transportrichtung weiterbewegt.

Um sicherzustellen, dass die Beschichtung 1a der Materialbahn 1 ausreichend getrocknet ist und nur noch geringe Restfeuchte aufweist, wird der Wassergehalt der Beschichtung 1a indirekt in einer Messkammer 3 gemessen, die gegenüber der Umgebung weitestgehend abgedichtet ist. Durch diese Messkammer wird die Materialbahn 1 während ihres Transports bewegt, wobei hierzu Rollen 6 vorgesehen sind.

Zur Durchführung der Messung des Wassergehalts der Beschichtung 1a wird die Messkammer 3 zunächst mit Messkammerluft AI gespült bzw. befüllt. Die Messkammerluft AI weist am Anfang eines Zeitintervalls IN, das nach dem Befüllvorgang beginnt, eine vorgegebene (d.h. vorbekannte) relative Luftfeuchtigkeit HU und eine vorgegebene (d.h. vorbekannte) Temperatur TP auf. Die Luftfeuchtigkeit HU und die Temperatur TP können beispielsweise mittels einer Sensorik (nicht gezeigt) in der Zuluft zur Messkammer 3 gemessen worden sein. Ebenso können der weiter unten beschriebene Feuchtigkeitssensor 4 bzw. der weiter unten beschriebene Temperatursensor 5 zur Messung der vorgegebenen relativen Luftfeuchtigkeit HU bzw. der vorgegebenen Temperatur TP genutzt werden.

Die Befüllung der Messkammer 3 ist in Fig. 1 durch den Richtungspfeil P1 angedeutet. Als Messkammerluft AI kann gegebenenfalls die Raumluft um die Messkammer 3 genutzt werden, sofern gewährleistet ist, dass die Temperatur und Luftfeuchtigkeit dieser Raumluft konstant sind. Nichtsdestotrotz kann die der Messkammer zugeführte Luft auch vorkonditioniert werden, damit sie die erwünschte Luftfeuchtigkeit und die erwünschte Temperatur aufweist. Die Messkammerluft AI kann beispielsweise eine Temperatur von 20 °C und eine relative Luftfeuchtigkeit von 5 % aufweisen.

Nach dem Befüllen der Messkammer 3 wird die Messkammerluft AI für einen bestimmten Zeitraum bei gleichzeitig leichter Umluft (ungefähr 5 m/s) in der Messkammer gehalten. Parallel erfolgt der Transport der Materialbahn 1 durch die Messkammer 3. Anschließend wird die Messkammerluft AI aus der Messkammer 3 ein oder mehrmals in vorbestimmten Zeitabständen entnommen, wie durch den Richtungspfeil P2 angedeutet ist. Bei jeder Entnahme wird mittels eines Feuchtigkeitssensors 4 die relative Luftfeuchtigkeit HU' der Messkammerluft AI und mittels eines Temperatursensors 5 die Temperatur TP' der Messkammerluft AI gemessen. Das Zeitintervall IN stellt dabei die Zeitspanne ab der Befüllung der Messkammer mit Messkammerluft bis zur letzten Messung der relativen Luftfeuchtigkeit HU' und der Temperatur TP' dar. Gegebenenfalls kann die Temperatur TP' auch nur bei der letzten Entnahme der Messkammerluft AI am Ende des Zeitintervalls IN gemessen werden. Vorzugsweise bleibt die Temperatur der Messkammerluft AI innerhalb des Zeitintervalls IN konstant, d.h. die vorgegebene Temperatur TP verändert sich nicht und entspricht der gemessenen Temperatur TP' am Ende des Zeitintervalls IN. Die kann z.B. dadurch erreicht werden, dass der Anfang des Zeitintervalls IN so gewählt wird, dass sich ein Temperaturgleichgewicht eingestellt hat. Ferner kann ggf. eine Heiz- und/oder Kühleinrichtung vorgesehen sein, um die Temperatur in der Messkammer 3 konstant zu halten

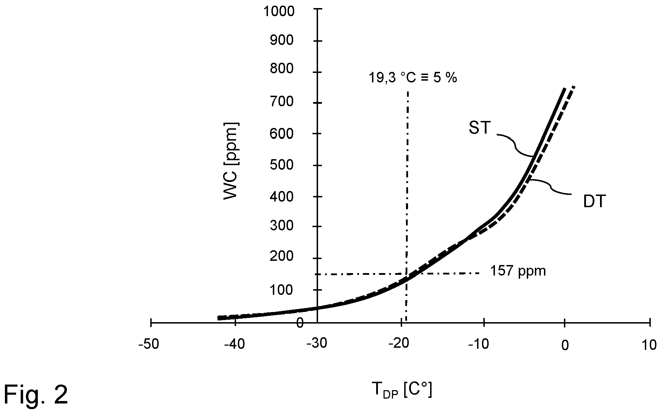

Im Rahmen der Erfindung macht man sich die Erkenntnis zunutze, dass die gemessene bzw. die gemessenen Luftfeuchtigkeiten HU' Aufschluss darüber geben, wie hoch der Wassergehalt der Beschichtung 1a ist. In der hier beschriebenen Ausführungsform nutzt man zur Bestimmung dieses Wassergehalts die Sorptionsisotherme und die Desorptionsisotherme der entsprechenden Beschichtung für die Temperatur TP' der Messkammerluft AI, die am Ende des Zeitintervalls IN vorliegt. Fig. 2 zeigt ein Diagramm, welches beispielhaft den Verlauf einer Sorptionsisotherme ST und Dessorptionsisotherme DT zeigt, die mittels eines Modells unter Verwendung der Gleichung (3.1.13) der Druckschrift [2] berechnet wurden. Als Beschichtung wurde die Substanz mit den Parametern aus Tabelle 5.4 der Druckschrift [2] zugrunde gelegt. Diese Substanz wird in Beschichtungen für Anoden verwendet. Als Temperatur TP' der umgebenden Luft wurden 22 °C angenommen.

Die Sorptionsisotherme ST der Fig. 2 repräsentiert den Gleichgewichtszustand zwischen dem Wassergehalt der Substanz und dem Wassergehalt der umgebenden Luft im Falle von vorangegangener Sorption von Wassermolekülen in die Substanz aus der umgebenden Luft (sog. Sorptionsgleichgewicht). In diesem Gleichgewichtszustand stellt sich eine bestimmte Luftfeuchte in der Luft ein, mithilfe derer mit bekannter Lufttemperatur der Taupunkttemperatur T_{DP} berechnet werden kann, welcher über die Sorptionsisotherme ST mit dem Wassergehalt (d.h. der Restfeuchte) WC der Substanz korreliert ist. Da die Temperatur der umgebenden Luft bekannt ist, entspricht die Taupunkttemperatur einer relativen Luftfeuchtigkeit der umgebenden Luft und umgekehrt. Beispielhaft ist in Fig. 2 für eine Taupunktemperatur von 19,3 °C (entspricht einer relativen Luftfeuchtigkeit von 5 %) der damit korrelierte Wassergehalt der Substanz von 157 ppm angegeben.

Die Desoptionsisotherme DT der Fig. 2 zeigt den gleichen Zusammenhang wie die Sorptionsisotherme ST, jedoch für den Fall, dass sich der Gleichgewichtszustand zwischen dem Wassergehalt der Substanz und dem Wassergehalt der umgebenden Luft im Falle von vorangegangener Desorption von Wassermolekülen aus der Substanz in die umgebende Luft einstellt. In dem Beispiel der Fig. 2 entspricht die Desorptionsisotherme DT der betrachteten Substanz im Wesentlichen ihrer Sorptionsisotherme ST.

In der Ausführungsform der Fig. 1 wird die gemessene bzw. die gemessenen relativen Luftfeuchtigkeiten HU' in einer Auswerteeinheit 7 verarbeitet, welche daraus eine Feuchtigkeitsinformation HI ermittelt. Hierzu ist in der Auswerteeinheit 7 eine Sorptionsisotherme und eine Desorptionsisotherme für die Temperatur TP' am Ende des Zeitintervalls IN und die entsprechende Beschichtung 1a, wie z.B. die Sorptionsisotherme ST und die Desorptionsisotherme DT aus Fig. 2, hinterlegt. Das Zeitintervall IN ist dabei derart gewählt, dass sich der entsprechende Gleichgewichtszustand spätestens am Ende des Zeitintervalls eingestellt hat. Die Auswerteeinheit bestimmt dann aus der zuletzt gemessenen relativen Luftfeuchtigkeit HU', die mit einer Taupunkttemperatur verknüpft ist, den Wassergehalt der Beschichtung als Feuchtigkeitsinformation HI. Ist diese relative Luftfeuchtigkeit kleiner gleich der vorgegebenen relativen Luftfeuchtigkeit HU, nutzt sie hierzu den Zusammenhang aus der Sorptionsisotherme. Ist diese relative Luftfeuchtigkeit größer als die vorgegebene Feuchtigkeit HU, nutzt sie hierzu den Zusammenhang aus der Desorptionsisotherme.

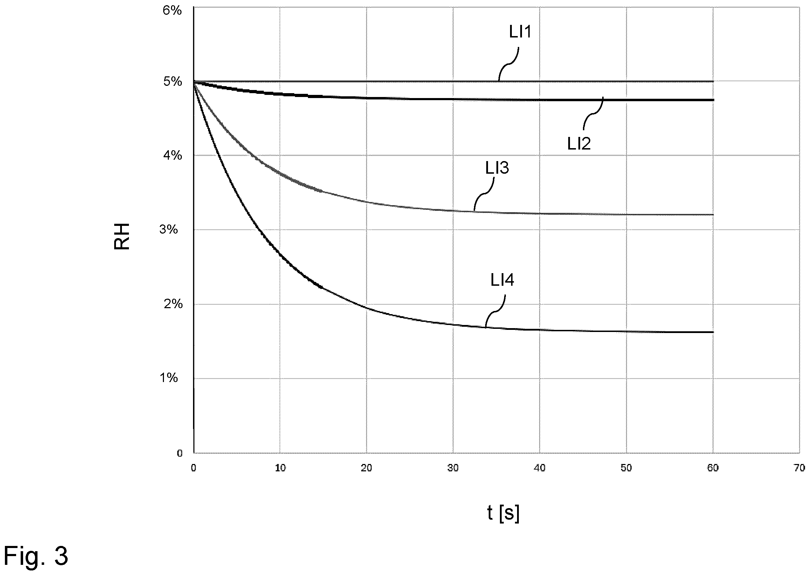

Fig. 3 zeigt beispielhaft die Veränderung der relativen Luftfeuchtigkeit RH in der Messkammer 3 bei fortschreitender Zeit t innerhalb des entsprechenden Zeitintervalls IN unter Verwendung der Sorptionsisotherme ST der Fig. 2. Dabei wird ein Szenario betrachtet, bei dem die Temperatur der Messkammerluft AI während des Zeitintervalls IN im Wesentlichen konstant bleibt. Als anfängliche vorgegebene relative Luftfeuchtigkeit HU der Messkammerluft AI wurden 5 % und als vorgegebene Temperatur TP der Messkammerluft AI wurden 22°C angenommen. Dies entspricht gemäß Fig. 2 einem Sorptionsgleichgewicht für einen Wassergehalt der Beschichtung von 157 ppm. Die einzelnen Linien LI1 bis LI4 der Fig. 3 wurden basierend auf einer Simulation bestimmt und beziehen sich auf die Substanz, welche der Sorptionsisotherme ST der Fig. 2 zugrunde liegt, jedoch mit unterschiedlichen Wassergehalten. Konkret entspricht die Linie LI1 einem Wassergehalt von 157 ppm, die Linie LI2 einem Wassergehalt von 150 ppm, die Linie LI3 einem Wassergehalt von 100 ppm und die Linie LI4 einen Wassergehalt von 50 ppm. Wie man aus Fig. 3 erkennt, stellt sich für die Linien L2 bis L4 nach Ablauf einer bestimmten Zeit ein Sorptionsgleichgewicht ein, denn die relative Luftfeuchtigkeit der Messkammerluft AI verändert sich nicht mehr. Für die Linie L1 lag das Sorptionsgleichgewicht bereits zum Zeitpunkt t = 0 vor.

Wie oben erwähnt, wird das Zeitintervall IN, d.h. der Zeitpunkt, zu dem die Luftfeuchtigkeit HU' gemäß Fig. 1 das letzte Mal gemessen wird, in der hier beschriebenen Ausführungsform so festgelegt, dass das Sorptionsgleichgewicht erreicht ist. Beispielsweise kann das Zeitintervall IN für das Szenario der Fig. 3 auf 50 Sekunden festgelegt werden, da nach Ablauf dieser Zeitspanne die relative Luftfeuchtigkeit RH weitestgehend konstant ist. Das Zeitintervall IN muss gegebenenfalls nicht fest vorgegeben sein, sondern kann auch in Abhängigkeit von der zeitlichen Veränderung der gemessenen relativen Luftfeuchtigkeit in der Messkammer 3 festgelegt werden. In diesem Fall wird die relative Luftfeuchtigkeit mehrmals im Zeitintervall IN gemessen. Dabei kann das Ende des Zeitintervalls und damit der letzte Messzeitpunkt daran gekoppelt sein, dass die Veränderung der gemessenen relativen Luftfeuchtigkeit (z.B. repräsentiert durch die Steigung der entsprechenden Linien aus Fig. 3) unter einer vorgegebenen Schwelle liegt.

In einer alternativen Variante gibt die Feuchtigkeitsinformation HI den Wassergehalt der Beschichtung nicht direkt an, sondern basierend auf einem Schwellwert. In diesem Fall liefert die entsprechende Sorptionsisotherme für die vorgegebene relative Luftfeuchtigkeit HU und die vorgegebene Temperatur TP der Messkammerluft AI den Schwellwert für den Wassergehalt der Beschichtung. Wird nunmehr festgestellt, dass die gemessene Luftfeuchtigkeit HU' der Messkammerluft AI anschließend abnimmt, kann hieraus geschlossen werden, dass der Wassergehalt der Beschichtung geringer als der Schwellwert ist. Steigt demgegenüber die gemessene relative Luftfeuchtigkeit an, kann hieraus geschlossen werden, dass der Wassergehalt der Beschichtung größer als der Schwellwert ist. Verändert sich die gemessene Luftfeuchtigkeit gegenüber der ursprünglich vorgegebenen Luftfeuchtigkeit nicht, kann hierausgeschlossen werden, dass der Wassergehalt der Beschichtung dem Schwellwert entspricht.

Die im Vorangegangenen beschriebenen Ausführungsformen der Erfindung weisen eine Reihe von Vorteilen auf. Insbesondere wird auf einfache Weise der Wassergehalt bzw. die Restfeuchte in der Beschichtung einer Materialbahn indirekt über die Messung der relativen Luftfeuchtigkeit der Luft in einer Messkammer bestimmt, ohne dass eine aufwändige Sensorik zur direkten Messung des Wassergehalts benötigt wird. Hierbei nutzt man den Umstand, dass sich zwischen dem Wassergehalt einer Beschichtung und dem Wassergehalt von umgebender Luft nach einer bestimmten Zeitspanne ein Gleichgewicht einstellt, wobei sich aus der Luftfeuchtigkeit der umgebenden Luft im Gleichgewicht auf den Wassergehalt bzw. die Feuchtigkeit der Beschichtung schließen lässt.

### Literaturverzeichnis

[1] M. Kosfeld et. al., Journal of Energy Storage 57 (2023) 106174
[2] Jochen Eser, Über das Stofftransport- und Sorptionsverhalten im Nachtrocknungsprozess von Elektroden für Li-Ionen-Batterien, Dissertation Karlsruher Institut für Technologie (2021)

### Bezugszeichenliste

- 1: beschichtete Materialbahn
- 1a: Beschichtung
- 2: Trocknerausgang
- 3: Messkammer
- 4: Feuchtigkeitssensor
- 5: Temperatursensor
- 6: Rollen
- 7: Auswerteeinheit
- AI: Messkammerluft
- HU: vorgegebene relative Luftfeuchtigkeit
- HU': gemessene relative Luftfeuchtigkeit
- TP: vorgegebene Temperatur
- TP': gemessene Temperatur
- IN: Zeitintervall
- T: Transportrichtung der Materialbahn
- P1, P2: Richtungspfeile
- ST: Sorptionsisotherme
- DT: Desorptionsisotherme
- WC: Wassergehalt
- T_{DP}: Taupunkttemperatur
- LI1, LI2, I3, LI4: Linien der relativen Luftfeuchtigkeit
- RH: relative Luftfeuchtigkeit
- t: Zeit

## Patentansprüche

1. Verfahren zur Analyse einer Beschichtung (1a) auf einer beschichteten Materialbahn (1) in Bezug auf darin enthaltene Feuchtigkeit, bei dem:
a) eine Messkammer (3) mit Messkammerluft (AI) befüllt wird, welche eine vorgegebene relative Luftfeuchtigkeit (HU) und eine vorgegebene Temperatur (TP) am Anfang eines Zeitintervalls (IN) aufweist, das nach dem Befüllen der Messkammer (3) beginnt;
b) durch die befüllte Messkammer (3) die beschichtete Materialbahn (1) transportiert wird und während des Transports der Materialbahn (1) ein oder mehrere Parameter der Messkammerluft (AI) umfassend die relative Luftfeuchtigkeit (HU') der Messkammerluft (AI) zumindest am Ende des Zeitintervalls (IN) gemessen werden;
c) aus der Veränderung der relativen Luftfeuchtigkeit der Messkammerluft (AI) innerhalb des Zeitintervalls (IN) mithilfe der vorgegebenen Temperatur (TP) der Messkammerluft (AI) und/oder mithilfe einer in Schritt b) gemessenen Temperatur (TP') der Messkammerluft (AI) am Ende des Zeitintervalls (IN) eine Feuchtigkeitsinformation (HI) ermittelt wird, welche den Wassergehalt in der Beschichtung (1a) der beschichteten Materialbahn (1) beschreibt.

2. Verfahren nach Anspruch 1, **dadurch gekennzeichnet, dass** die Temperatur der Messkammerluft (AI) innerhalb des Zeitintervalls (IN) im Wesentlichen konstant bleibt.

3. Verfahren nach Anspruch 1 oder 2, **dadurch gekennzeichnet, dass** sich die Feuchtigkeitsinformation (HI) aus zumindest einer Sorptionsisotherme (ST) und/oder zumindest einer Desorptionsisotherme (DT) ergibt, welche jeweils einen Gleichgewichtszustand zwischen dem Wassergehalt in der Beschichtung (1a) und dem Wassergehalt in umgebender Luft mit der vorgegebenen Temperatur (TP) und/oder der gemessenen Temperatur (TP') am Ende des Zeitintervalls (IN) repräsentieren, wobei die zumindest eine Sorptionsisotherme (ST) im Falle einer vor Erreichen des Gleichgewichtszustands stattgefundenen Sorption von Wasser in die Beschichtung (1a) aus der umgebenden Luft mit der vorgegebenen Temperatur (TP) und/oder der gemessenen Temperatur (TP') am Ende des Zeitintervalls (IN) die Abhängigkeit des Wassergehalts in der Beschichtung (1a) von der relativen Luftfeuchtigkeit der umgebenden Luft mit der vorgegebenen Temperatur (TP) und/oder der gemessenen Temperatur (TP') am Ende des Zeitintervalls (IN) im Gleichgewichtszustand wiedergibt und die zumindest eine Desorptionsisotherme im Falle einer vor Erreichen des Gleichgewichtszustands stattgefundenen Desorption von Wasser aus der Beschichtung (1a) in die umgebende Luft mit der vorgegebenen Temperatur (TP) und/oder der gemessenen Temperatur (TP') zumindest am Ende des Zeitintervalls (IN) die Abhängigkeit des Wassergehalts in der Beschichtung (1a) von der relativen Luftfeuchtigkeit der umgebenden Luft mit der vorgegebenen Temperatur (TP) und/oder der gemessenen Temperatur (TP') am Ende des Zeitintervalls (IN) im Gleichgewichtszustand wiedergibt.

4. Verfahren nach Anspruch 3, **dadurch gekennzeichnet, dass** in Schritt c) bei Abnahme der relativen Luftfeuchtigkeit der Messkammerluft (AI) ausgehend von der vorgegebenen relativen Luftfeuchtigkeit (HU) innerhalb des Zeitintervalls (IN) als zumindest ein Teil der Feuchtigkeitsinformation (HI) der Wassergehalt in der Beschichtung (1a) ermittelt wird, der sich aus der Sorptionsisotherme (ST) für die gemessene relative Luftfeuchtigkeit (HU') der Messkammerluft (AI) und die gemessene Temperatur (TP') der Messkammerluft (AI) am Ende des Zeitintervalls (IN) ergibt.

5. Verfahren nach Anspruch 3 oder 4, **dadurch gekennzeichnet, dass** in Schritt c) bei Zunahme der relativen Luftfeuchtigkeit der Messkammerluft (AI) ausgehend von der vorgegebenen relativen Luftfeuchtigkeit (HU) innerhalb des Zeitintervalls (IN) als zumindest ein Teil der Feuchtigkeitsinformation (HI) der Wassergehalt in der Beschichtung (1a) ermittelt wird, der sich aus der Desorptionsisotherme (4) für die gemessene relative Luftfeuchtigkeit (HU') der Messkammerluft (AI) und die gemessene Temperatur (TP') der Messkammerluft (AI) am Ende des Zeitintervalls (IN) ergibt.

6. Verfahren nach einem der Ansprüche 3 bis 5, **dadurch gekennzeichnet, dass** ein Schwellwert für den Wassergehalt in der Beschichtung (1a) festgelegt ist und die vorgegebene Luftfeuchtigkeit (HU) und die vorgegebene Temperatur (TP) der Messkammerluft (AI) derart eingestellt werden, dass sich aus der Sorptionsisotherme (ST) für die vorgegebene Luftfeuchtigkeit (HU) und die vorgegebene Temperatur (TP) der umgebenden Luft der Schwellwert ergibt, wobei als zumindest ein Teil der Feuchtigkeitsinformation (HI) ermittelt wird, dass der Wassergehalt in der Beschichtung (1a) kleiner gleich dem Schwellwert ist, wenn die relative Luftfeuchtigkeit der Messkammerluft (AI) ausgehend von der vorgegebenen Luftfeuchtigkeit (HU) innerhalb des Zeitintervalls (IN) abnimmt oder konstant bleibt, und dass Wassergehalt in der Beschichtung (1a) größer als der Schwellwert ist, wenn die relative Luftfeuchtigkeit der Messkammerluft (AI) ausgehend von der vorgegebenen relativen Luftfeuchtigkeit (HU) innerhalb des Zeitintervalls (IN) zunimmt.

7. Verfahren nach einem der vorhergehenden Ansprüche, **dadurch gekennzeichnet, dass** das Zeitintervall (IN) fest vorgegeben ist und vorzugsweise zwischen 10 Sekunden und 10 Minuten, besonders bevorzugt zwischen 20 Sekunden und 1 Minute, liegt.

8. Verfahren nach einem der Ansprüche 1 bis 6, **dadurch gekennzeichnet, dass** das Zeitintervall (IN) dann beendet wird, wenn die Veränderung der relativen Luftfeuchtigkeit der Messkammerluft (AI) unterhalb einer vorgegebenen Schwelle liegt.

9. Verfahren nach einem der vorhergehenden Ansprüche, **dadurch gekennzeichnet, dass** die Messkammerluft (AI) in der Messkammer (3) zirkuliert.

10. Verfahren nach einem der vorhergehenden Ansprüche, **dadurch gekennzeichnet, dass** die Messkammerluft (AI) gegenüber der Luft in der Umgebung der Messkammer (3) unter Überdruck steht.

11. Verfahren nach einem der vorhergehenden Ansprüche, **dadurch gekennzeichnet, dass** die Messkammer (3) eine Länge in Transportrichtung (T) der Materialbahn (1) zwischen 20 cm und 40 cm und/der eine Höhe in Richtung senkrecht zur Ebene der Materialbahn (1) zwischen 3 cm und 5 cm und/oder eine Breite senkrecht zur Transportrichtung der Materialbahn (1) zwischen 200 mm und 1600 mm aufweist.

12. Verfahren nach einem der vorhergehenden Ansprüche, **dadurch gekennzeichnet, dass** die beschichtete Materialbahn (1) eine Elektrodenmaterialbahn für eine Batterie ist.

13. Verfahren nach einem der vorhergehenden Ansprüche, **dadurch gekennzeichnet, dass** das Verfahren nach einem Trocknungsprozess der beschichteten Materialbahn (1) durchgeführt wird.

14. Vorrichtung zur Analyse einer Beschichtung (1a) auf einer beschichteten Materialbahn (1) in Bezug auf darin enthaltene Feuchtigkeit, wobei die Vorrichtung zur Durchführung eines Verfahrens eingerichtet ist, bei dem:
a) eine Messkammer (3) mit Messkammerluft (AI) befüllt wird, welche eine vorgegebene relative Luftfeuchtigkeit (HU) und eine vorgegebene Temperatur (TP) am Anfang eines Zeitintervalls (IN) aufweist, das nach dem Befüllen der Messkammer (3) beginnt;
b) durch die befüllte Messkammer (3) die beschichtete Materialbahn (1) transportiert wird und während des Transports der Materialbahn (1) ein oder mehrere Parameter der Messkammerluft (AI) umfassend die relative Luftfeuchtigkeit (HU') der Messkammerluft (AI) zumindest am Ende des Zeitintervalls (IN) gemessen wird;
c) aus der Veränderung der relativen Luftfeuchtigkeit der Messkammerluft (AI) innerhalb des Zeitintervalls (IN) mithilfe der vorgegebenen Temperatur (TP) der Messkammerluft (AI) und/oder mithilfe einer in Schritt b) gemessenen Temperatur (TP') der Messkammerluft (AI) am Ende des Zeitintervalls (IN) eine Feuchtigkeitsinformation (HI) ermittelt wird, welche den Wassergehalt in der Beschichtung (1a) der beschichteten Materialbahn (1) beschreibt.

15. Vorrichtung nach Anspruch 14, **dadurch gekennzeichnet, dass** die Vorrichtung zur Durchführung eines Verfahrens nach einem der Ansprüche 2 bis 13 eingerichtet ist.
